# EUROPEAN PATENT APPLICATION

(11) **EP 4 668 281 A1**
(43) Date of publication of application: **24.12.2025**
(21) Application number: 24843564.6
(22) Date of filing: 19.07.2024
(51) Int. Cl.: G16C 20/70, G16C 20/30, G16C 20/40, G16C 20/50, G16C 20/80, G06N 20/00

(54) **CHEMICAL REACTION PREDICTION SYSTEM AND ITS CONTROL METHOD, AND LEARNING METHOD OF THE CHEMICAL REACTION PREDICTION SYSTEM**

(30) Priority: 19.07.2023 KR 20230093644
(71) Applicant: LG Management Development Institute Co., Ltd., Seoul 07336 (KR)
(72) Inventor: HORMAZABAL, Rodrigo, Incheon 21022 (KR); BERTENS, Paul, Seoul 07788 (KR); HAN, Se Hui, Seoul 07665 (KR)
(74) Representative: BCKIP Part mbB
(86) International application number: PCT/KR2024/010505
(87) International publication number: WO 2025/018853

(57) **Abstract**

The present invention relates to a chemical reaction prediction system and a control method thereof, and a learning method of the chemical reaction prediction system. More specifically, the present invention relates to a chemical reaction prediction system performing forward reaction prediction based on an electron flow and a control method thereof, and a learning method of the chemical reaction prediction system.

## Description

### [Technical Field]

Various embodiments disclosed in the present document a chemical reaction prediction system, a control method thereof, and a learning method of a chemical reaction prediction system, and more particularly, to a chemical reaction prediction system which performs forward reaction prediction based on an electron flow, a control method thereof, and a learning method of the chemical reaction prediction system.

### [Background Art]

With the development of artificial intelligence, there is a rapid increase in cases where excellent results are achieved through artificial intelligence technology in various fields.

In particular, in the field of natural science, attempts are being made continuously to solve various scientific problems using artificial intelligence technology. For example, in the field of chemistry, research is actively being conducted using artificial intelligence technology to predict the results of chemical reactions between molecules or to design new molecules.

In this regard, organic synthesis is one of the important challenges in drug development and materials science, and predicting the results of the chemical reactions is very important in designing new molecules.

In the related art, a sequence-based model using SMILES strings is utilized to predict the results of the chemical reactions, but since conversion using SMILES strings does not naturally express molecular structures in a graph form, there is a limitation in that it does not sufficiently reflect the graph structure of molecules.

To solve these problems, various graph-based approaches have been proposed to represent molecules as graphs. Graph representations can accurately represent the structure of molecules and provide intuitive information for interpreting and predicting chemical reaction mechanisms. In particular, studies utilizing a graph neural network (GNN) are actively being conducted, which have the advantage of accurately modeling interactions between atoms in molecules.

### [Disclosure]

### [Technical Problem]

The present invention provides a chemical reaction prediction system that performs forward reaction prediction based on electron flow, a control method thereof, and a learning method of the chemical reaction prediction system.

Furthermore, the present invention provides a chemical reaction prediction system that performs an electron-flow inspired graph diffusion model for interpretable forward reaction prediction, a control method thereof, and a learning method of the chemical reaction prediction system.

The present invention provides a chemical reaction prediction system capable of performing a good understanding of molecular structures and accurately predicting a chemical reaction between molecular structures based on the understanding, a control method thereof, and a learning method of the chemical reaction prediction system.

In addition, the present invention provides a chemical reaction prediction system capable of predicting results of various types of chemical reactions by understanding the chemical reaction mechanism, a control method thereof, and a learning method of the chemical reaction prediction system.

More specifically, the present invention provides a chemical reaction prediction model based on electron flow using graph diffusion, in which both input and output structures are formed in graph space.

Furthermore, the present invention provides a learning method of a chemical reaction prediction model capable of performing a good understanding of the chemical reaction mechanism and generating more accurate and predicted results of interpretable chemical reactions.

### [Technical Solution]

A chemical reaction prediction method performed by cooperation of a memory and a processor according to various embodiments disclosed in the present document may include: receiving information related to a plurality of molecular structures as input to an encoder; acquiring an embedding vector corresponding to the plurality of molecular structures using the information related to the plurality of molecular structures in an embedding layer of the encoder; performing an attention operation related to interaction between atoms of the plurality of molecular structures in a multi-head self-attention layer and updating the embedding vector based on the operation; storing the embedding vector updated through the updating in the memory and inputting the updated embedding vector stored in the memory to a decoder; performing bond prediction and atom prediction predicted as a chemical reaction of the plurality of molecular structures using the updated embedding vector in the decoder; and acquiring a final chemical reaction product predicted from the chemical reaction of the plurality of molecular structures using a result of the bond prediction and a result of the atom prediction.

In an embodiment, the acquiring of the chemical reaction product may include sampling an initial chemical reaction product using the result of the bond prediction and the result of the atom prediction, stabilizing the sampled initial chemical reaction product through a diffusion feedback process, and acquiring the final chemical reaction product stabilized through the diffusion feedback process.

In an embodiment, the chemical reaction prediction method may further include acquiring a molecular graph for the plurality of molecular structures by converting atoms into nodes and bonds between atoms into edges based on the plurality of molecular structures, in which the plurality of molecular structures may include a first molecular structure and a second molecular structure, and the acquiring of the molecular graph may further include acquiring a first molecular graph including nodes and edges corresponding to the first molecular structure by converting atoms constituting the first molecular structure into nodes and a bond relationship between atoms constituting the first molecular structure into edges using a pre-specified graph transformation algorithm, and acquiring a second molecular graph including nodes and edges corresponding to the second molecular structure by converting atoms constituting the second molecular structure into nodes and a bond relationship between atoms constituting the second molecular structure into edges using the pre-specified graph transformation algorithm.

In an embodiment, the information related to the plurality of molecular structures may include information on the nodes and edges corresponding to the first molecular structure and information on the nodes and edges corresponding to the second molecular structure, and the embedding vector may include at least one of information on an atom type, an atom charge, the number of hydrogens, the number of radical electrons, and a degree of the node corresponding to the first molecular structure and the node corresponding to the second molecular structure.

In an embodiment, in the updating of the embedding vector, different biases may be added to an attention score operated by the multi-head self-attention layer according to a bond type between the nodes constituting the first molecular graph and the second molecular graph.

In an embodiment, the bond type may include a single bond type, a double bond type, a triple bond type, and an aromatic bond type.

In an embodiment, the chemical reaction prediction method may further include extracting, using the nodes and edges corresponding to the first molecular graph and the nodes and edges corresponding to the second molecular graph, at least one of an adjacency matrix, a bond type matrix, a shortest paths matrix, and K-hop neighbors corresponding to each of the first molecular graph and the second molecular graph, in which the adjacency matrix may include information on direct connection between the nodes constituting the first molecular graph and the second molecular graph, the bond type matrix may include information on a bond type between the nodes constituting the first molecular graph and the second molecular graph, the shortest paths matrix may include information on a shortest path length between the nodes constituting the first molecular graph and the second molecular graph, and the K-hop neighbors may include information on neighboring nodes within a K step for each of the nodes constituting the first molecular graph and the second molecular graph.

In an embodiment, in the updating of the embedding vector, an output vector of the multi-head self-attention layer may be added to a feed-forward neural network layer, in the feed-forward neural network layer, the output vector of the multi-head self-attention layer may be updated using at least one of the adjacency matrix, the bond type matrix, the shortest paths matrix, and the K-hop neighbors,, and the output vector of the feed-forward neural network layer may be specified as the updated embedding vector.

In an embodiment, the bond prediction may be performed by performing a dot-product using the updated embedding vector, and the dot-product may be performed for each vector corresponding to each atom pair of atoms corresponding to the updated embedding vector.

In an embodiment, the performing of the bond prediction may include acquiring an inner product value for each of plurality of bond types for each atom pair based on the dot-product, and the plurality of bond types may be related to at least one of a single bond, a double bond, a bond formation, a bond collapse, and no change.

In an embodiment, the performing of the bond prediction may further include generating a probability distribution for each of the plurality of bond types for each atom pair using the inner product value according to the dot-product, and acquiring a transformation matrix that predicts a change in a bonded state of each atom pair using the probability distribution.

In an embodiment, in the generating of the probability distribution, for each atom pair, a Softmax function may be applied to the inner product values acquired for the plurality of bond types for each atom pair to generate the probability distribution for the plurality of bond types for each atom pair.

In an embodiment, the performing of the atom prediction may further include generating an atomic characteristic probability distribution of each atom corresponding to the updated embedding vector using a Softmax output layer, and predicting atomic characteristics of the atoms corresponding to the updated embedding vector using the probability distribution, and the atomic characteristics may include charge states of the atoms changeable during a chemical reaction process of the plurality of molecule structures.

In an embodiment, in the diffusion feedback process, each bond transformation of the initial chemical reaction product may be repeatedly evaluated and an unstable bond may be removed or changed.

In an embodiment, in the diffusion feedback process, in order to predict a change in a bonded state of the initial chemical reaction product, predicted bond transformation may be evaluated at each repeatedly performed step using a transformation probability matrix and a target transformation matrix, and the final chemical reaction result may be generated using an interpolation factor.

A learning method for a chemical reaction prediction method performed by cooperation of a memory and a processor according to various embodiments disclosed in the present document may include: receiving information related to a plurality of molecular structures as input to an encoder; acquiring a molecular graph using atoms as nodes and bonds as edges based on the plurality of molecular structures; acquiring an embedding vector corresponding to the molecular graph using the information related to the plurality of molecular structures in an embedding layer of the encoder; performing an attention operation related to interaction between atoms of the plurality of molecular structures in a multi-head self-attention layer and updating the embedding vector based on the operation; storing the embedding vector updated through the updating in the memory and inputting the updated embedding vector stored in the memory to a decoder; performing bond prediction and atom prediction predicted as a chemical reaction of the plurality of molecular structures using the updated embedding vector in the decoder; acquiring a final chemical reaction product predicted from the chemical reaction of the plurality of molecular structures using a result of the bond prediction and a result of the atom prediction; calculating a loss function between the final chemical reaction product and label data including an actually bonded state and an atomic state corresponding to the plurality of molecular structures; and optimizing at least one parameter of the encoder and the decoder to minimize the loss function.

A chemical reaction prediction system according to various embodiments disclosed in the present document may include: a memory; an encoder; a decoder; and at least one a processor, in which the encoder receives information related to a plurality of molecular structures, and acquires a molecular graph using atoms as nodes and bonds as edges based on the plurality of molecular structures, acquires an embedding vector corresponding to the molecular graph in an embedding layer of the encoder, and performs an attention operation related to interaction between atoms of the plurality of molecular structures in a multi-head self-attention layer of the encoder and updates the embedding vector based on the operation, the processor stores the embedding vector updated through the updating in the memory, and inputs the updated embedding vector stored in the memory to the decoder, and the decoder performs bond prediction and atom prediction predicted as a chemical reaction of the plurality of molecular structures using the updated embedding vector, and acquires a final chemical reaction product predicted from the chemical reaction of the plurality of molecular structures using a result of the bond prediction and a result of the atom prediction.

A program stored on a computer-readable recording medium, executable by one or more processes on an electronic device according to another aspect of the present invention may include instructions to execute: receiving information related to a plurality of molecular structures as input to an encoder; acquire a molecular graph using atoms as nodes and bonds as edges based on the plurality of molecular structures; acquiring an embedding vector corresponding to the molecular graph in an embedding layer of the encoder; performing an attention operation related to interaction between atoms of the plurality of molecular structures in a multi-head self-attention layer and updating the embedding vector based on the operation; storing the embedding vector updated through the updating in a memory and inputting the updated embedding vector stored in the memory to a decoder; performing bond prediction and atom prediction predicted as a chemical reaction of the plurality of molecular structures using the updated embedding vector in the decoder; and acquiring a final chemical reaction product predicted from the chemical reaction of the plurality of molecular structures using a result of the bond prediction and a result of the atom prediction.

### [Advantageous Effects]

As described above, according to the chemical reaction prediction system, the control method thereof, and the learning method of a chemical reaction prediction system according to the present invention, by modeling the movement of electrons through the graph structure of molecules, it is possible to better understand the chemical reaction mechanism and accurate prediction of the results of the chemical reaction.

In addition, according to the chemical reaction prediction system, the control method thereof, and the learning method of a chemical reaction prediction system according to the present invention, it is possible to provide the chemical reaction prediction model that can understand the chemical reaction mechanism well and accurately predict the results of the chemical reaction, thereby allowing the user to reduce the time and cost required for experiments. Through this, the research and development cost can be reduced and the time to market a new product can be shortened.

Meanwhile, according to the chemical reaction prediction system, according to the chemical reaction prediction system, the control method thereof, and the learning method of the chemical reaction prediction system according to the present invention, by performing both the input of the molecular structure and the output of the predicted product in the graph space, it is possible to perform the forward reaction prediction regardless of the SMILES permutation and order.

Furthermore, according to the chemical reaction prediction system, the control method thereof, and the learning method of the chemical reaction prediction system according to the present invention, by simultaneously sampling multiple interdependent transformations that occur in parallel within the molecular graph, it is possible to secure the consistency between the transformations.

Furthermore, according to the chemical reaction prediction system, the control method thereof, and the learning method of the chemical reaction prediction system according to the present invention, in order to solve problems that may occur due to a symmetrical structure, by breaking the symmetry and forming the valid output structure by including the noise or sampling mechanisms, it is possible to prevent the occurrence of the invalid configuration.

### [Description of Drawings]

FIG. 1 is a conceptual diagram for describing a chemical reaction prediction system and a control method thereof according to the present invention, and an answer generation system to which a learning method of the chemical reaction prediction system is applied.
FIGS. 2A to 2F are conceptual diagrams for describing a chemical reaction prediction model according to the present invention.
FIG. 3 is a flow chart for describing a learning method of the chemical reaction prediction model according to the present invention.
FIGS. 4A to 4D are conceptual diagrams for describing the chemical reaction prediction model according to the present invention.
FIGS. 5A to 5E, 6, and 7 are conceptual diagrams for describing examples of use in an answer system to which the chemical reaction prediction model according to the present invention is applied.

### [Mode for Disclosure]

Hereinafter, embodiments disclosed in this specification will be described in detail with reference to the accompanying drawings, but the same or similar components will be denoted by the same reference numerals independent of the drawing numerals, and an overlapping description of the same or similar components will be omitted. In addition, the terms "module" and "unit" for components used in the following description are used only to easily make the disclosure. Therefore, these terms do not have meanings or roles that distinguish from each other in themselves. Further, in describing the embodiments disclosed in this specification, if it is determined that a detailed description of related known technologies may obscure the gist of the embodiments disclosed in this specification, the detailed description thereof is omitted. In addition, it is to be understood that the accompanying drawings are provided only for easy understanding of embodiments disclosed in this specification, and the technical idea disclosed in this specification is not limited by the accompanying drawings, but includes all the modifications, equivalents, and substitutions included in the spirit and the scope of the present invention.

The terms including ordinal numbers such as 'first' and 'second' may be used to describe various components, but these components are not limited by these terms. The terms are used to distinguish one component from another component.

It is to be understood that when one component is referred to as being "connected to" or "coupled to" another component, one component may be connected directly to or coupled directly to another component or be connected to or coupled to another component with the other component interposed therebetween. On the other hand, it is to be understood that when one component is referred to as being "connected directly to" or "coupled directly to" another component, it may be connected to or coupled to another component without the other component interposed therebetween.

Singular forms include plural forms unless the context clearly indicates otherwise.

It will be further understood that the terms "include" or "have" used in the present specification specify the presence of features, numerals, steps, operations, components, parts mentioned in the present specification, or combinations thereof, but do not preclude the presence or addition of one or more other features, numerals, steps, operations, components, parts, or combinations thereof.

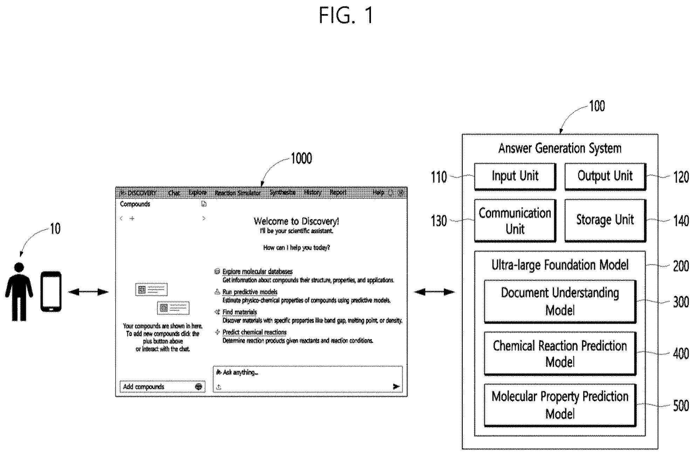

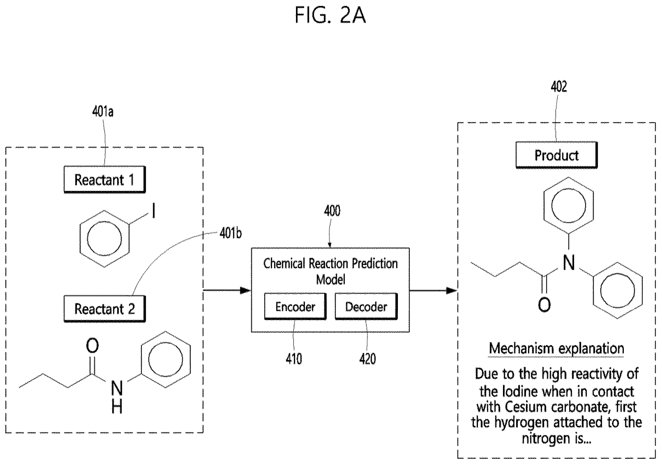

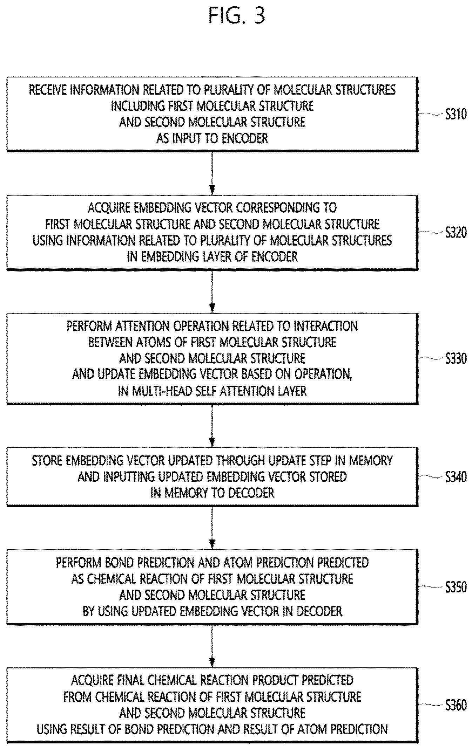

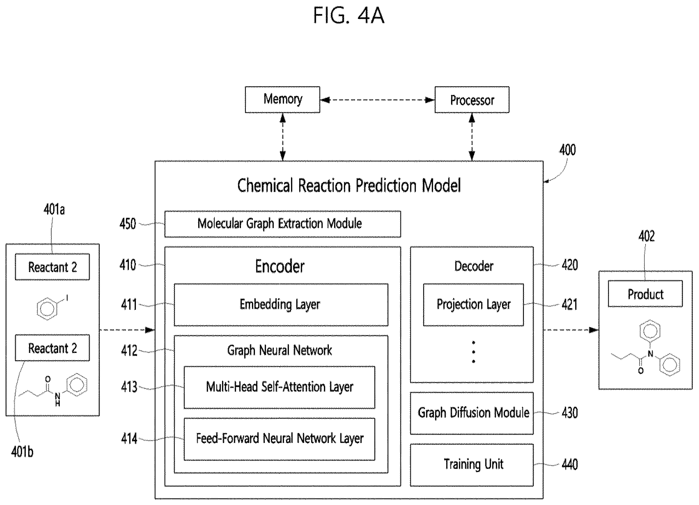

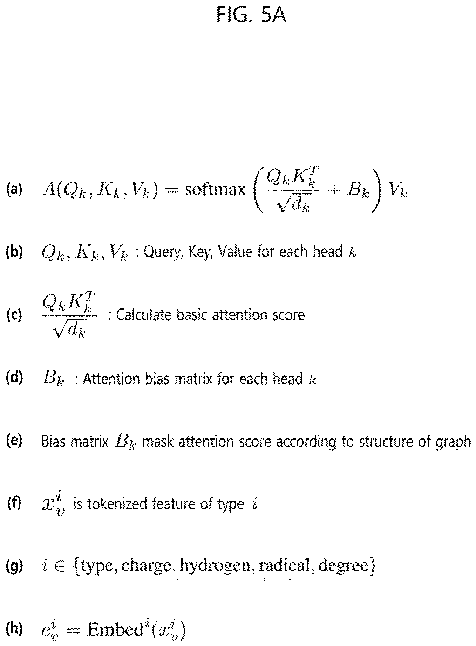

Hereinafter, the present invention will be described in more detail with reference to the attached drawings. FIG. 1 is a conceptual diagram for describing a chemical reaction prediction system and a control method thereof according to the present invention, and an answer generation system to which a learning method of the chemical reaction prediction system is applied. FIGS. 2A to 2F are conceptual diagrams for describing a chemical reaction prediction model according to the present invention, and FIG. 3 is a flow chart for describing a learning method of the chemical reaction prediction model according to the present invention. Moreover, FIGS. 4A to 4D are conceptual diagrams for describing the chemical reaction prediction model according to the present invention, and FIGS. 5A to 5E, 6, and 7 are conceptual diagrams for describing examples of use in an answer system to which the chemical reaction prediction model according to the present invention is applied.

The chemical reaction prediction system and control method thereof according to the present invention, and the learning method of the chemical reaction prediction system may be usefully utilized in various situations. The prediction of chemical reactions is utilized in various ways, and for example, it can be usefully utilized in research for designing new materials or developing new drugs. In this regard, organic synthesis is one of the important tasks in the development of new drugs and materials science, and predicting the results of the chemical reaction is very important in designing new molecules. The present invention provides a system, a control method, and a learning method for predicting a chemical reaction between molecular structures by converting a molecular structure into a graph and predicting a chemical reaction based on the graph. The chemical reaction prediction system and control method thereof according to the present invention, and the learning method of the chemical reaction prediction system are implemented based on a "chemical reaction prediction model", and for the convenience of description, the system, the method, and the learning method are not named separately, but are uniformly referred to as a "chemical reaction prediction" model.

Meanwhile, the chemical reaction prediction model according to the present invention may be applied to various industries and services, and for example, may be usefully utilized by being applied to an answer generation system based on a language model. Recently, along with the development of deep learning technology, generative AI technology has been attracting attention recently. More specifically, a generative AI model may generate new data in various forms such as text, images, and voice from given data, and this provides a different level of application potential from simply classifying or predicting existing data. The chemical reaction prediction model according to the present invention may also be applied to such an answer generation system, and may be usefully utilized in various fields requiring chemical reaction prediction, such as research for designing new materials or developing new drugs.

Hereinafter, the answer generation system to which the chemical reaction prediction model according to the present invention can be applied will be briefly examined with reference to FIG. 1. The answer generation system according to FIG. 1 may include various prediction and analysis models, and may be a system that uses the models to generate property prediction results of molecular structures or to design molecules having characteristics desired by a user. In addition, the answer generation system according to the present invention may be a system that generates chemical reaction prediction results between new types of molecules and/or multiple molecules. Furthermore, the answer generation system according to the present invention may be a system that generates prediction results of transformations of existing materials and synthesis of various materials (e.g., new materials, polymer materials, nanomaterials, composite materials, organic materials, pharmaceutical materials, or the like).

The answer generation system according to the present invention includes an ultra-large foundation model (or a large foundation artificial intelligence model, or a generative artificial intelligence model), and the present invention aims to increase the efficiency of natural science research by minimizing the risk of research failure. This answer generation system may also be referred to as an answer generation platform based on the ultra-large foundation model. However, the "ultra-large foundation model" may also be referred to as a generative model, a foundation model, or a large language model (LLM).

Meanwhile, as illustrated in FIG. 1, an answer generation system 100 may include at least one of an input unit 110, an output unit 120, a communication unit 130, a storage unit 140, and an ultra-large foundation model 200. Here, the ultra-large foundation model 200 may also be referred to as a foundation model, and the foundation model may mean an ultra-large AI core base model trained with a massive dataset.

Although not illustrated, the answer generation system 100 may include one or more processors, which may include one or more general-purpose processors and/or one or more special-purpose processors (e.g., a digital signal processor, a tensor processing unit (TPU), a graphics processing unit (GPU), a neural network processing unit (NPU), an application-specific integrated circuit, an application-specific integrated circuit (ASIC), or the like). The one or more processors may be configured to execute instructions stored in (or included in) the storage unit 140, computer-readable instructions, and/or other instructions described herein. The answer generation system and method may enable the memory and at least one processor to cooperate to perform the data processing described below. The processor may perform a series of operations and data processing using data and information stored in the memory. In this case, the memory may be a component of the storage unit 140.

Meanwhile, the input unit 110 may be configured as a means for data input and may be configured in various types. For example, the input unit 110 may be configured to receive user input. The input unit 110 may be configured to receive user input from a user terminal 10. Here, "receiving input" may mean receiving an input signal (or selection signal) corresponding to the user's input based on the input being made by the user through the input unit configuration provided in the user terminal 10.

The input unit 110 may also be referred to as a user interface module. The input unit 110 may include a touch screen, a computer mouse, a keyboard, a keypad, a touch pad, a trackball, a joystick, a voice recognition module, or other similar devices. However, the present invention does not place any limitation on the type of the input unit 110. In addition, the input unit 110 in the present invention does not necessarily mean a hardware means, and may be understood as a passage for receiving input from a user.

Here, the user input may include documents, text, images (or videos), voice, or the like. In this case, the answer generation system 100 may further include a module that converts voice into text.

Next, the output unit 120 may output information through an output unit configuration (e.g., a display unit, a touch screen, a speaker, or the like) equipped in a user terminal 10 linked to the answer generation system 100. For example, the output unit 120 may output a page (or service page) 1000 linked to the answer generation system 100 to the display unit of the user terminal 10. In addition, the output unit 120 does not necessarily mean a hardware means, and may be understood as a passage for outputting results to the user.

Next, the communication unit 130 may be connected to the user terminal 10, the server (e.g., a central server, an external server, or the like), a device, and at least one network through a wireless or wired network, and may be configured to receive or transmit overall data and information necessary for the operation of the answer generation system 100.

Here, the user terminal 10 may include at least one of a mobile phone, a smart phone, a notebook computer, a laptop computer, a slate PC, a tablet PC, an ultrabook, a desktop computer, a digital broadcasting terminal, personal digital assistants (PDA), a portable multimedia player (PMP), navigation, a wearable device (e.g., a smartwatch, a smart glass, and a head mounted display (HMD)), and the like.

Furthermore, the communication unit 130 may support various communication methods according to the communication standards of the communicating device.

For example, the communication unit 130 may be configured to communicate with a communication target using at least one of wireless LAN (WLAN), Wireless-Fidelity (Wi-Fi), Wireless Fidelity (Wi-Fi) direct, digital living network alliance (DLAN), Wireless Broadband, World Interoperability for Microwave Access (WiMAX), High Speed Downlink Packet Access (HSDPA), High Speed Uplink Packet Access (HSUPA), Long Term Evolution (LTE), Long Term Evolution-Advanced (LTE-A), 5th Generation (5G) Mobile Telecommunication, Bluetooth^{™} Radio Frequency Identification (RFID), Infrared Data Association; IrDA), Ultra-Wideband (UWB), ZigBee, Near Field Communication (NFC), Wi-Fi Direct, and Wireless Universal Serial Bus (USB) technologies.

Meanwhile, the storage unit 140 serves to store various data related to the present invention and may include one or more non-transitory computer-readable storage media that may be read and/or accessed by at least one of the one or more processors 140.

The one or more computer-readable storage media may include volatile and/or nonvolatile storage components such as optical, magnetic, organic or other memory or disk storage devices. In some examples, the storage unit 140 may be implemented using a single physical device (e.g., one optical, magnetic, organic, or other memory or disk storage device), while in other examples, the storage unit 140 may be implemented using two or more physical devices.

The storage unit 140 may include computer-readable instructions and additional data. The storage unit 140 may include storage necessary to perform at least some of methods, scenarios and techniques described herein and/or at least some of the functions of the devices and networks.

Furthermore, at least a portion of the storage unit 140 may be a cloud storage or a cloud server. The storage unit 140 may store at least some of data corresponding to the user input received from the input unit 110 and training data.

That is, it can be understood that the storage unit 140 is sufficient as a space where information necessary for the operation of the answer generation system 100 is stored, and there are no restrictions on the physical space.

Meanwhile, the ultra-large foundation model 200 may be configured to generate property prediction results of molecular structures or to design molecules having desired characteristics by the user. In addition, the ultra-large foundation model 200 may generate prediction results of new types of molecules and/or chemical reactions between multiple molecules, or predicted results of transformations of existing materials and synthesis of various materials (e.g., new materials, polymer materials, nanomaterials, composite materials, organic materials, pharmaceutical materials, or the like).

In this regard, the ultra-large foundation model 200 may include at least one of a document understanding model 300, a chemical reaction prediction model 400, and a molecular property prediction model 500.

The document understanding model 300 can extract various types of content that satisfy preset content criteria from documents (e.g., papers, books, patent documents, reports, or the like). More specifically, the document understanding model 300 may be a model trained to understand structured data, unstructured data, linguistic data (or linguistic elements), and non-linguistic data (or non-linguistic elements) included in a document, and extract various contents (or data) and knowledge based on the understood contents.

Here, the preset content criteria may be set in various ways and may be determined according to the purpose or use of the answer generation system 100 according to the present invention.

For example, when the utilization purpose of the answer generation system 100 is chemistry, bio, new materials, new substances, and new drug development, the document understanding model 300 may be trained to understand and extract the contents related to the chemistry, the bio, the new materials, the new substances, and the new drug development from an analysis target document.

In this case, the preset content criteria may include the contents related to the molecular structures that are related to at least one of the chemistry, the bio, the new materials, the new substances, and the new drug development. Here, the document understanding model 300 may extract the contents related to the chemistry, the bio, the new materials, the new substances, and the new drug development from the analysis target document based on the preset content criteria.

In this specification, for convenience of description, the preset content criteria are described as being related to at least one of the chemistry, the bio, the new materials, the new substances, and the new drug development, but are not necessarily limited thereto.

Based on the preset content criteria, the document understanding model 300 may extract at least one of text, a molecular structure, a formula, a chart, a table, and an image, which satisfy the preset content criteria, from the analysis target document.

For example, the document understanding model 300 may understand the chemical structure of the molecular structure included in the analysis target document 20, and extract the molecular structure by transforming the molecular structure into a SMILES string expression based on the result of understanding. In addition, the document understanding model 300 may understand the chemical structure of the molecular structure and perform graph transformation corresponding to the molecular structure based on the result of understanding.

Meanwhile, in the present invention, receiving the "molecular structure" may be understood as receiving information that may specify a molecule. In this case, the information that may specify the molecular structure may be in various forms such as a molecular structure formula, a molecular graph, a chemical formula, a molecular structure formula based on a SMILES notation, a molecular structure image, etc.

In addition, the document understanding model 300 may understand text 23 related to a molecular structure 21 among the texts included in the analysis target document 20, and may extract the text 23 as text data 23 related to the molecular structure 21.

Furthermore, the document understanding model 300 may recognize rows and columns that constitute a table 24 related to the molecular structure 21 from the analysis target document 20 and extract structured data 25 by converting the recognized rows and columns into the structured data 25 in a format such as HTML or Excel.

Furthermore, the document understanding model 300 may extract relationship information (or relationship) between the molecular structures included in the document.

As described above, the document understanding model 300 may extract various types of data included in a document by converting the data into data (e.g., machine-readable data) in a form that the ultra-large foundation model 200 may understand. The data extracted using the document understanding model 300 may be sorted in units of pages or documents and stored in the storage unit 140 (or memory). In the present invention, the document understanding model 300 may also be called a deep document understanding model.

Next, the chemical reaction prediction model 400 may be configured to predict a chemical reaction between input molecular structures (or compounds or reactants) and output (or generate) the predicted results (or products) of chemical reaction. For example, as illustrated in FIG. 2A, when information on molecular structures 401a and 401b of a specific compound is input, the chemical reaction prediction model 400 may predict a product 402 formed by the chemical reaction between molecular structures 401a and 401b of the specific compound.

The chemical reaction prediction model 400 according to the present invention may model the movement of electrons through a graph structure of molecules. The chemical reaction prediction model 400 according to the present invention may be an electron flow-based prediction model using graph diffusion that may better understand the chemical reaction mechanism and accurately predict the results of the chemical reaction. The chemical reaction prediction model 400 may express both input and output in a graph space. The chemical reaction prediction model 400 may train the graph transformation of the chemical reaction to output reaction products and/or byproduct prediction results based on the graph structure of the input compound. The chemical reaction prediction model 400 in the present invention may also be called an electron flow model, an electron flow-based forward reaction prediction model, or an electron-flow inspired graph diffusion model for interpretable forward reaction prediction model.

Meanwhile, the chemical reaction prediction model 400 described above may include at least one of an encoder 410 and a decoder 420.

The encoder 410 according to the present invention may be configured to extract features of each atom and bond through a graph representation of a molecular structure and generate an embedding vector based on the extracted features. In addition, by performing an attention operation related to interaction between the atoms of the plurality of molecular structures through a neural network, the characteristics of the molecular structure may be better understood. The decoder 420 may perform bond prediction and atom prediction, which are predicted as the chemical reaction of the plurality of molecular structures, using the embedding vector acquired from the encoder 410. The chemical reaction prediction model 400 may perform a diffusion feedback process to stabilize the chemical reaction products according to the bond prediction and atom prediction, thereby generating a final chemical reaction product. The composition and detailed process of the chemical reaction prediction model 400 according to the present invention will be described in more detail later.

Meanwhile, the chemical reaction prediction model 400 according to the present invention may use not only the structural information of molecules but also various types of chemical information such as reaction conditions, catalysts, and temperatures in order to predict more accurate chemical reactions. Such chemical information may include structural data (e.g., molecular structure graph) of molecules and descriptive data (e.g., text data). Since the chemical reactions are affected by various factors such as structural changes of molecules, reaction mechanisms, reaction conditions, and reaction environments, more accurate predictions can be possible only when these elements are comprehensively considered. Meanwhile, such chemical information may exist in various literature, textbooks, papers, patent literatures, articles, and academic journals, and the chemical reaction prediction model 400 according to the present invention may perform more accurate chemical reaction predictions by integrating such chemical information.

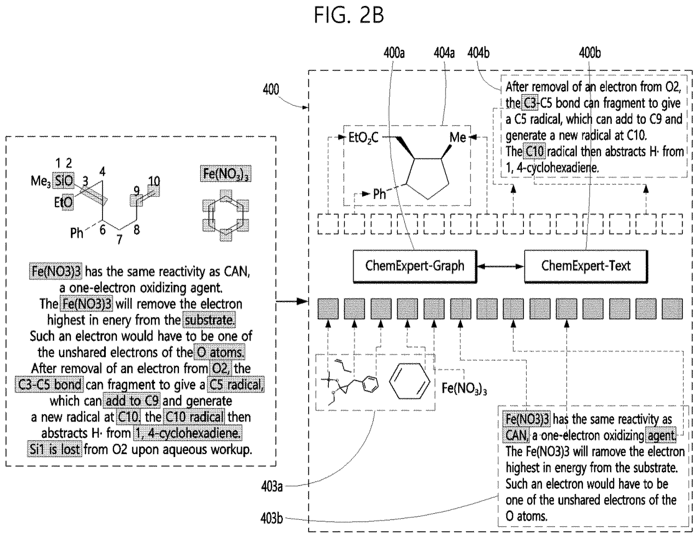

To this end, as illustrated in FIG. 2B, the chemical reaction prediction model 400 may include at least one of a first module 400a and a second module 400b. Here, the first module 400a may also be named as a "ChemExpert-Graph" module, a graph module, a graph processing module, a graph model, a graph processing model, etc., and the second module 400b may also be called as a "ChemExpert-Text" module, a text module, a text processing module, a text model, a text processing model, etc.

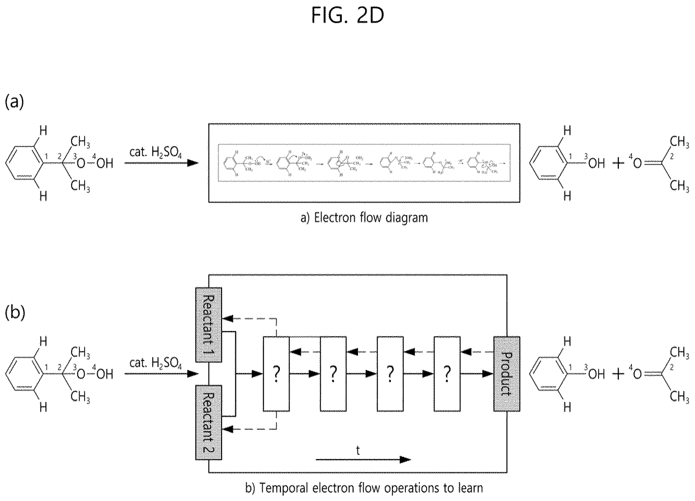

The first module 400a may receive a molecular (or chemical) structure as input and predict a graph-based chemical reaction. For example, referring to (a) of FIG. 2D, an example of the chemical reaction may be confirmed. As illustrated in (b) of FIG. 2D, the first module 400a may include a plurality of layers 400a-1 to predict the graph-based chemical reaction. More specific details regarding the plurality of layers 400a-1 will be described later.

A molecular structure 403a (or molecular structure formula) input to the first module 400a is converted into a molecular graph in the form of a graph, and atoms in the molecular graph may be expressed as nodes and bonds may be expressed as edges.

The molecular structure 403a may correspond to at least one of data extracted from a document 403 including a molecular structure 411 using the document understanding model 300, or information extracted through the storage unit 140 (or memory).

The first module 400a may analyze changes in structural characteristics of a molecule based on the input molecular graph, predict a chemical reaction path and a product to be generated as the results of the chemical reaction, and output the predicted chemical reaction path and product.

In an embodiment, the first module 400a may analyze structural changes of a molecule based on the molecular graph, and predict a process in which a specific bond is separated and a new bond is formed.

In another embodiment, the first module 400a may analyze the interaction between the atoms in the molecule based on the molecular graph, and predict radical formation and bond changes that may occur at each step.

That is, the first module 400a may be configured to receive the molecular graph as input, and output the predicted chemical reaction path and a product 404a based on the molecular graph.

Next, the second module 400b may be configured to process text data 403b to understand and predict a reaction mechanism. In this case, the text data 403b may correspond to at least one of data extracted from a document including the molecular structure 403a using the document understanding model 300, or information extracted through the storage unit 140 (or memory) related to the molecular structure 411. The second module 400b may be a model that has pre-trained vast data related to the chemical reaction.

In an embodiment, the text data 403b input to the second module 400b is data including a description of the molecular structure 403a, and may include at least one of chemical reaction conditions, chemical reaction mechanism (or reaction path), and chemical characteristics of the molecular structure 403a.

The second module 400b may analyze the input text data 403b to understand and predict the chemical reaction mechanism. More specifically, the second module 400b may analyze the input text data 403b and output at least one of the chemical reaction conditions, chemical reaction mechanisms, and chemical characteristics that are predicted based on the text data 403b.

In an embodiment, the second module 400b may analyze the text data 403b using a natural language processing (NLP) technology and extract at least one text of the chemical reaction conditions, chemical reaction mechanisms (or reaction paths), chemical characteristics, and experimental data included in the text data 403b.

In another embodiment, the second module 400b may predict the chemical reaction mechanism (e.g., how a specific catalyst or condition affects the reaction) based on the text extracted through the analysis of the text data 403b, and output the predicted chemical reaction mechanism and chemical characteristics.

The second module 400b may analyze the information related to the chemical reaction prediction, which is related to the plurality of molecular structures, from the text data.

The chemical reaction prediction model 400 may combine the output data 404a of the first module 400a and the output data 404b of the second module 400b to output the predicted results (e.g., product, chemical reaction path, chemical reaction mechanism, etc.) of the final chemical reaction.

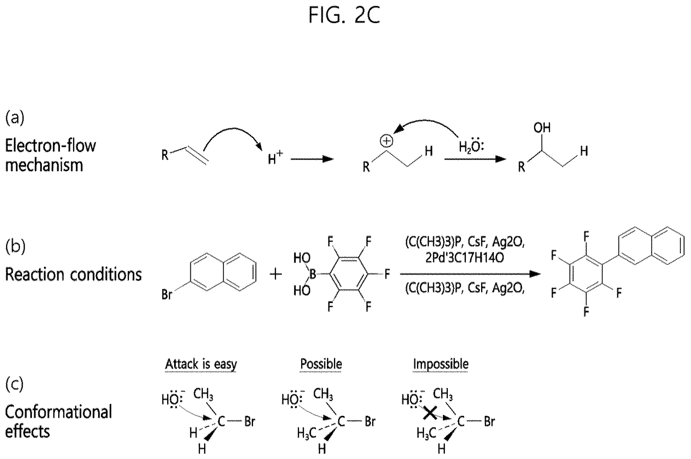

In an embodiment, as illustrated in (a) to (c) of FIG. 2C, the chemical reaction prediction model 400 may generate electron flow, reaction conditions, and structural effects of a molecular structure (or chemical structure) using the output data output from the first module 400a and the second module 400b. In this case, the electron flow, the reaction conditions, and the structural effects may be expressed together as the graph and text, the molecular structure reflecting the position before and after the electron moves may be generated, or the molecular structure of the product generated according to the reaction conditions may be generated.

That is, the chemical reaction prediction model 400 can make more accurate predictions than using only a single data source by fusing the output data 404a and 404b output from the first module 400a and the second module 400b, respectively, and may enable users to intuitively recognize various elements related to chemical reactions.

In addition, the chemical reaction prediction model 400 may verify the chemical reaction products predicted through the first module 400a using the output data analyzed in the second module 400b. That is, the second module 400b may acquire at least one of the chemical reaction conditions, chemical reaction mechanism, and chemical characteristics analyzed based on the text data 403b. The chemical reaction prediction model 400 may verify whether the chemical reaction products predicted and acquired in the first module 400a match the experimental data or theoretical expectations based on the data analyzed in the second module 400b.

Meanwhile, the chemical reaction prediction model 400 according to the present invention is characterized by performing the chemical transformation based on the molecular graph, and for understanding, the configuration of the molecular structure graph will be schematically described.

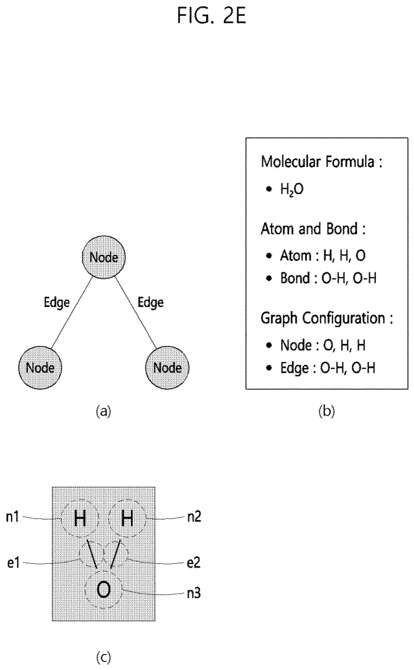

(a) of FIG. 2E illustrates nodes and edges, and in the molecular graph, atoms may be expressed as nodes and bonds between atoms may be expressed as edges. For example, as illustrated in (b) of FIG. 2E, a water molecule has a molecular formula of "H2O". In this case, atoms are composed of H, H, and O. In this case, the number of nodes is three, and as illustrated in (c) of FIG. 2E, the atoms H, H, and O may be expressed as nodes n1, n2, and n3, respectively. In addition, there may be two bonds between atoms, O-H and O-H, and their bonds may be expressed as edges e1 and e2, as illustrated in (c) of FIG. 2E. In this way, when converting the molecular structure into the molecular graph, the unique positional relationship and topological relationship of the molecular structure may be preserved, so it is possible to implement more accurate prediction. In the present invention, the molecular structure to be predicted is converted into the molecular graph and input to the encoder 410 to predict the chemical reaction.

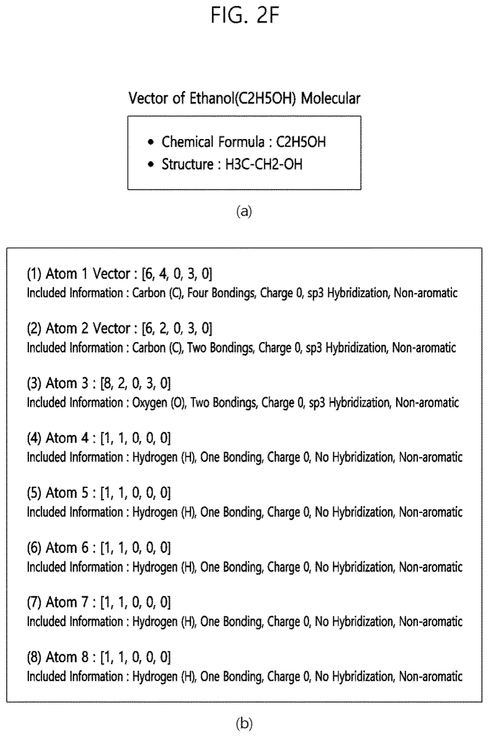

Meanwhile, in the present invention, the encoder 410 may embed the molecular structure into a vector using the molecular graph. In this case, briefly describing the vector, as illustrated in (a) of FIG. 2F, an ethanol (C2H5OH) molecule may represent ethanol through a vector having a specific dimension, as illustrated in (b) of FIG. 2F. For example, a vector corresponding to each atom of ethanol may be expressed as illustrated in (b) of FIG. 2F. In this case, the dimension of the vector and the included information may be set in various ways. For example, (b) of FIG. 2F illustrates a five-dimensional vector, and each vector may include atom type, binding information, charge information, hybridization information, directionality information, etc. Meanwhile, although not illustrated, the encoder 410 may embed a vector for the binding information of the molecule and utilize the embedded vector for analysis.

Hereinafter, a method of predicting a chemical reaction based on a molecular graph and acquiring the chemical reaction products according to the chemical reaction prediction model 400 according to the present invention will be examined in more detail. The description below may be performed in the first module(ex: the encoder 410) illustrated in FIG. 2B.

The chemical reaction prediction and organic synthesis are one of the important challenges in new drug development and/or material science, and it is very important to predict the results of the chemical reaction in designing new molecules, which may greatly contribute to shortening a product development cycle in various industrial fields. Conventionally, a sequence-based model using a SMILES string was utilized to predict chemical reactions and/or retrosynthetic path. The SMILES strings encode chemical structures (or molecules or molecular structures) into ASCII strings, and thus, may be processed as text data through natural language processing technology.

However, the SMILES strings do not match the natural graph representation of molecules, so learning efficiency is low, and chemically invalid transformations (or modifications) may be generated. In particular, in the case of the chemical reactions, the transformations in the SMILES space may not directly correspond to valid molecular graphs, so chemically infeasible outputs may occur. That is, there is a limitation that such transformation does not always lead to a valid modification in the graph space, which is a more natural representation of molecules.

To solve this problem, various graph-based (or centric) approaches that represent molecules as graphs have been proposed. The graph representations may accurately represent the structure of molecules, increase the interpretability of chemical reaction mechanisms, and facilitate more accurate chemical reaction predictions based on electron flow theory.

Accordingly, the present invention provides a chemical reaction prediction model based on an electron flow using graph diffusion that models the movement of electrons through the graph structure of molecules to better understand the chemical reaction mechanisms and accurately predict the results of the chemical reaction. The prediction method according to the present invention may also be named an electron-flow inspired graph diffusion model for interpretable forward reaction prediction method.

The chemical reaction prediction model described in the present invention operates in the graph space for both inputs and outputs using the graph diffusion, which maintains invariance of the permutation and order of the SMILES strings while providing more interpretable transformations to users. The chemical reaction prediction model according to the present invention may train a conversion process starting from an initial graph set rather than starting from random graph sampling and leading to a final product.

Hereinafter, the present invention will be described in more detail with reference to the accompanying drawings.

As illustrated in FIG. 4A, the chemical reaction prediction model 400 according to the present invention may include the encoder 410, the decoder 420, a graph diffusion module 430, and a training unit 440. As illustrated, the chemical reaction prediction model 400 may perform a series of data processing according to the present invention by cooperation of a memory and at least one processor. Various types of information may be stored in the memory. For example, the memory may store various types of information such as information input to the chemical reaction prediction model 400, products generated according to a series of data processing in the chemical reaction prediction model 400, information on intermediate products, and information on the final product. The processor may cooperate with each component of the chemical reaction prediction model to allow the process for chemical reaction prediction according to the present invention to proceed in each component and a neural network(a neural network layer).

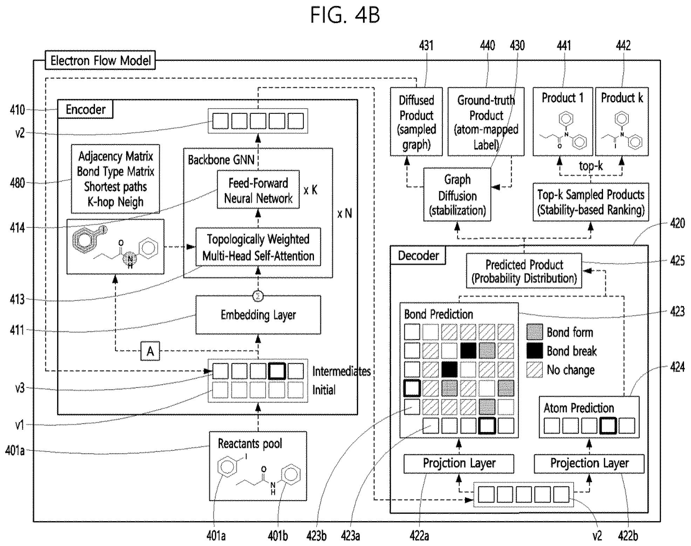

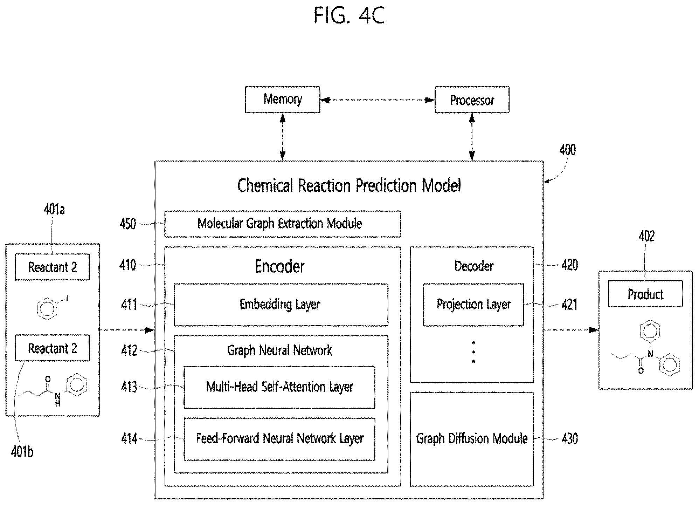

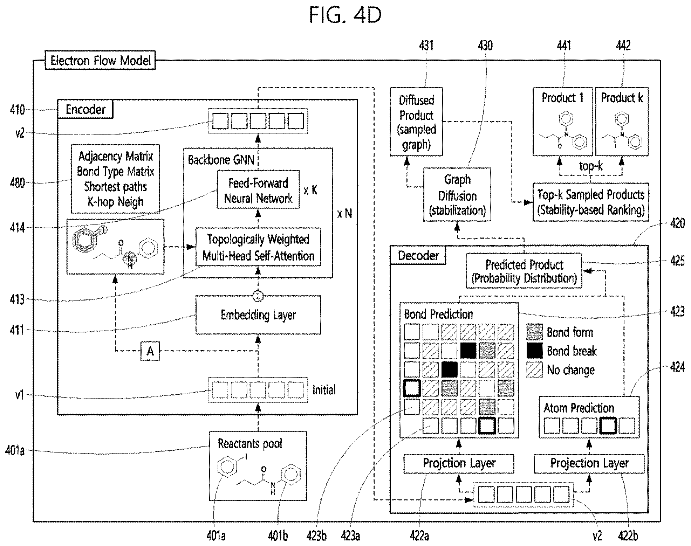

The chemical reaction prediction model 400 according to the present invention has the training unit 440 and a ground-truth product as illustrated in FIGS. 4A and 4B in the training step, but after the training is completed, the training unit and the ground-truth product may be excluded as illustrated in FIGS. 4C and 4D.

The encoder 410 according to the present invention may be configured as a backbone encoder. The encoder 410 according to the present invention is configured as an attention-based graph neural network (GNN) and may perform relative position encoding based on the shortest path between the nodes (atoms). Meanwhile, the diffusion process described in the present invention does not depend on the backbone network and may also be implemented with various encoders.

The encoder 410 according to the present invention may include an embedding layer 411 and graph neural networks (GNN) 412 as illustrated. The graph neural network may be the attention-based graph neural network or the backbone neural network.

Meanwhile, the graph neural networks 412 may include a multi-head self-attention layer 413 and a feed-forward neural network layer. The multi-head self-attention layer may also be named a topologically weighted multi-head self-attention layer.

The embedding layer 411 is configured to receive information related to the plurality of molecular structures and convert the received information into the embedding vector. The embedding layer 411 may be configured to generate a vector including atoms of the molecular structure and a bond relationship between the atoms. The graph neural networks 412 may receive the embedding vector embedded in the embedding layer 411, perform an attention operation related to the interaction between the atoms of the plurality of molecular structures, and update the embedding vector based on the operation. In this case, the embedding layer may receive the molecular graph and acquire the embedding vector based on the molecular graph.

Meanwhile, the node features contained in the embedding vector output by the embedding layer 411 may include an atom type, an atom charge, the number of implicit hydrogens, and the number of radical electrons. The embedding layer 411 may generate the embedding vector including information that may be used in the encoder by encoding the nodes according to the molecular graph. In this way, the molecular graph may be completely reconstructed into each molecule, and the degree of the node may also be tokenized as an input function and used to measure the consistency between the bond prediction and the predicted atomic degree later.

Features of an atom v may be represented as (f), (g), and (h) of FIG. 5A, and may be tokenized and embedded through the embedding layer 411.

As illustrated in (g) of FIG. 5A, the features of the atom v are tokenized and embedded through the embedding layer 411, and the tokenized features of type i according to (f) of FIG. 5A may have features as illustrated in (g) of FIG. 5A. i is an index indicating a type of atomic features, and may belong to a set of type, charge, hydrogen, radical, and degree. i may mean at least one of the features of type, charge, hydrogen, radical, and degree. Here, the type may mean an atom type (e.g., carbon, oxygen, etc.), the charge may mean a formal charge of an atom, the hydrogen may mean the number of implicit hydrogens, the radical may mean the number of radical electrons of the atom, and the degree may mean a degree of a node (the number of other atoms bonded to the corresponding atom). These features are embedded through the embedding layer 411, as illustrated in (h) of FIG. 5A, to form the initial embedding of the atom. The initial embedding of the atom v may be an average of all the embeddings.

The embedding layer 411 may perform embedding for the plurality of molecules based on the molecular graph. The molecular graph for the molecular structure may be stored in a memory, a dataset, a database, etc. In contrast, the molecular graph for the molecular structure may be extracted by a molecular graph extraction module 450. The chemical reaction prediction model 400 may further include the molecular graph extraction module 450 or may cooperate with the molecular graph extraction module 450. Furthermore, the molecular graph extraction module 450 may be included as a component of the encoder 410.

The molecular graph may maintain a plurality of molecular structures by expressing the atoms as the nodes and the bonds between the atoms as the edges.

The plurality of molecular structures may include a first molecular structure and the second molecular structure, and the first and second molecular structures may be named reactants. In FIG. 4A, they correspond to reference numerals 401a and 401b.

The molecular graph extraction module 450 may acquire the molecular graph for the plurality of molecular structures by converting the atoms into the nodes and the bond relationship between the atoms into the edges based on the plurality of molecular structures using a pre-specified graph transformation algorithm.

The molecular graph extraction module 450 may acquire a first molecular graph including the nodes and edges corresponding to the first molecular structure by converting the atoms constituting the first molecular structure into the nodes and the bond relationship between the atoms constituting the first molecular structure into the edges. Similarly, the molecular graph extraction module 450 may acquire the second molecular graph including the nodes and edges corresponding to the second molecular structure by converting the atoms constituting the second molecular structure into the nodes and the bond relationship between the atoms constituting the second molecular structure into the edges using the pre-specified graph transformation algorithm.

In the embedding layer 411 of the encoder 410, the embedding vector corresponding to the plurality of molecular structures may be acquired using the information related to the plurality of molecular structures. In this case, the information related to the plurality of molecular structures may include the information on the nodes and edges corresponding to the first molecular structure and the information on the nodes and edges corresponding to the second molecular structure. The information related to the molecular structure may be the molecular graph extracted by the molecular graph extraction module 450 or the information extracted from the molecular graph.

The embedding vector may include at least one of the information on the atom type, the atom charge, the number of hydrogens, the number of radical electrons, and the degree of the node corresponding to the first molecular structure and the node corresponding to the second molecular structure. Furthermore, the embedding vector may be configured to further include the information on the bond relationship between the atoms of the first and second molecular structures.

Meanwhile, when the reactants to be predicted, i.e., the plurality of molecular structures (e.g., the first molecular structure and the second molecular structure 401a and 401b) are specified, the chemical reaction prediction model 400 may extract at least one of an adjacency matrix, a bond type matrix, shortest paths, and K-hop neighbors corresponding to the first molecular graph and the second molecular graph, respectively, using the nodes and edges corresponding to the first molecular graph and the nodes and edges corresponding to the second molecular graph. The time point at which the information 480 is extracted is not limited and may vary.

The adjacency matrix may include information on direct connections between the nodes constituting the first molecular graph and the second molecular graph.

The bond type matrix may include information on the bond types between the nodes constituting the first molecular graph and the second molecular graph.

The shortest paths matrix may include information on shortest path lengths between the nodes constituting the first molecular graph and the second molecular graph.

The K-hop neighbors may include information on neighboring nodes within K steps for each of the nodes constituting the first molecular graph and the second molecular graph.

At least one 480 of the adjacency matrix, the bond type matrix, the shortest paths, and the K-hop neighbors extracted in this manner may be stored in the memory. At least one of the adjacency matrix, bond type matrix, shortest paths, and K-hop neighbors stored in the memory may be input to at least one of the embedding layer 411, the graph neural networks 412, the multi-head self-attention layer 413, and a feed-forward neural network 414.

In the embedding layer 411, when the embedding vector v1 corresponding to the plurality of molecular structures (or reactants, for example, the first molecular structure and the second molecular structure 401a and 401b) is acquired, the acquired embedding vector v1 may be input to the graph neural networks 412. In particular, the embedding vector v1 may be input to the multi-head self-attention layer 413 of the graph neural networks.

The multi-head self-attention layer 413 may perform an attention operation related to the interaction between the atoms of the plurality of molecular structures using the embedded embedding vector v1 based on the molecular graph, and update the embedding vector based on the operation. In this case, the embedding vector v1 to be updated may be a vector output from the embedding layer.

The multi-head self-attention layer 413 may perform global attention-based pooling. In order to consider the molecular graph topology, the multi-head self-attention layer 413 may add a bias to the attention weight score derived from the shortest paths between node pairs. This may act as a relative position encoding between the atoms. As a result, it may be free from the spectrum-based encoding problem (e.g., lack of sign invariance for Laplace matrix eigenvectors) because the distance measurement between the nodes is not absolute. Such encoding has high computation efficiency, and may take into account the entire graph topology while allowing the global message delivery. In this case, the calculation time of the shortest paths may vary.

The global attention-based pooling is one that considers the entire topology of the graph, and the multi-head self-attention layer 413 may globally consider the correlation between each node of the graph by performing the global attention-based pooling.

For example, when the dimension of the input vector from the embedding layer is assumed to be 128, and the number of heads in the multi-head attention is assumed to be 8, a 16-dimensional sub-vector obtained by dividing 128-dimensional input vectors by the number of heads which is 8 may be input to each head.

Each head of the multi-head self-attention layer 413 is configured to independently calculate attention values for 16-dimensional sub-vectors by applying the preset attention mechanism. For example, a first head may mainly reflect information of adjacent nodes in the molecular graph, and a second head may mainly reflect information of a specific bond type in the molecular graph. The 16-dimensional attention vectors calculated from a plurality of heads of the multi-head self-attention layer 413, for example, 8 heads, respectively, may be combined again to become a 128-dimensional vector. This combined vector may have the same dimension as the embedding vector output from the embedding layer 411, but may include richer information by integrating various types of information extracted from each head. In the present invention, this may be expressed as the updated embedding vector.

Meanwhile, in order to reflect the shortest paths between the nodes in the weight, as described above, in the present invention, the shortest paths between all pairs of nodes in the molecular graph may be calculated. The shortest paths may be calculated by a preset algorithm. In the multi-head self-attention layer 413, the relative positions between the nodes may be encoded using the calculated shortest path information. This encoding may be performed based on the shortest path distance between the nodes. The multi-head self-attention layer 413 may use the shortest path information as the bias when calculating the attention weight score between the nodes. For example, the multi-head self-attention layer 413 may adjust the attention weight in such a way that the closer the shortest path distance between two nodes, the higher the attention weight, and the farther the shortest path distance between two nodes, the lower the attention weight. The multi-head self-attention layer 413 may add the bias derived from the shortest path between the node pairs to the attention weight score.

The multi-head self-attention layer 413 encodes each node of the molecular graph using the attention weight adjusted based on the shortest paths in this way, which enables the encoding that considers the topology of the entire molecular graph.

In addition, in the multi-head self-attention layer 413, the bias may be added to the attention score according to the bond type (e.g., single, double, triple, and aromatic bonds).

For example, in the multi-head self-attention layer 413, different biases may be added to the attention score calculated by the multi-head self-attention layer according to the bond type between the nodes constituting the first molecular graph and the second molecular graph.

Here, the bond type may include a single bond type, a double bond type, a triple bond type, and an aromatic bond type.

This may mean that, instead of a single bias matrix for all heads, each head has its own bias matrix. For example, the bias of the shortest path may be added to the first head, and the bias according to the single bond may be added to the second head. The remaining heads may use a mask that focuses on the global attention between the atoms or molecules. In the present invention, in the multi-head self-attention layer 413, such various bias matrices may be added to each layer according to the multi-head. According to the present invention, by adding the bias matrix to each layer corresponding to the multi-head, the attention score of each head may emphasize pieces of information masked by the bias matrix. For example, when the bias matrix according to the bond type is added, the head may be masked according to the bond type and receive information only from neighbors connected by a specific bond type. For example, a specific head may receive information from the nodes connected only by the single bond.

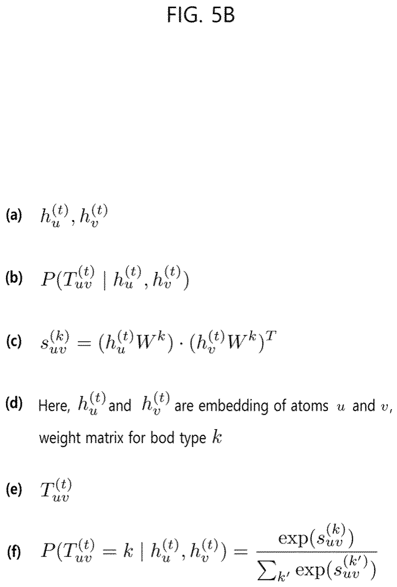

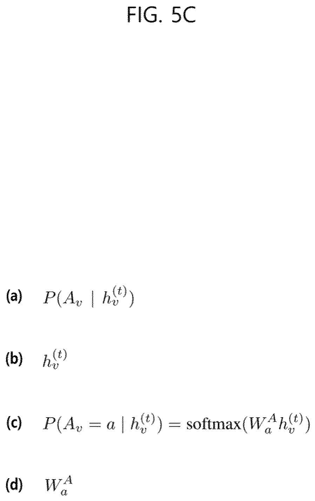

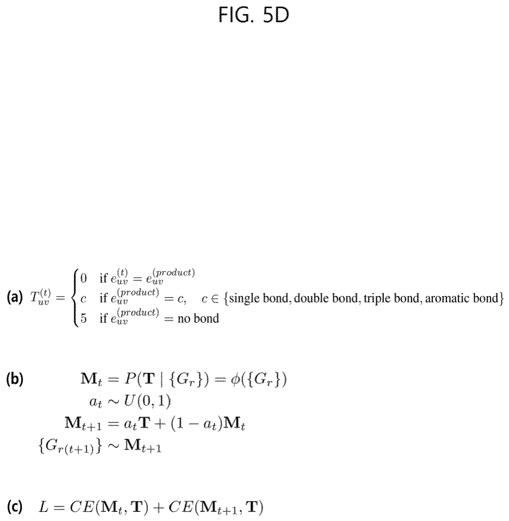

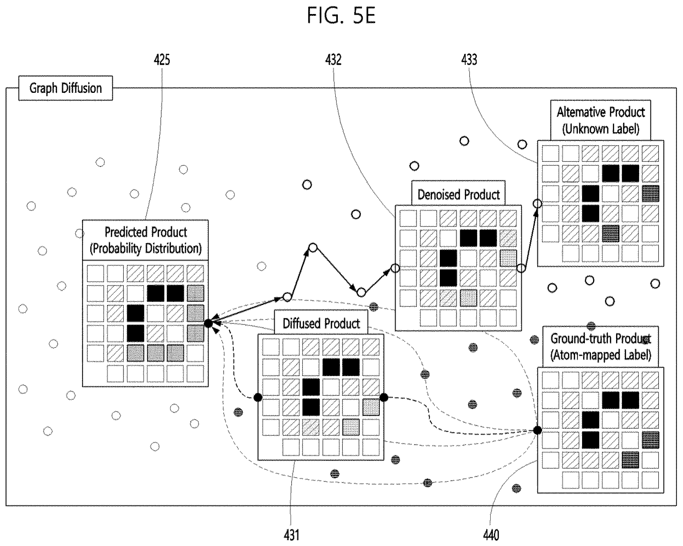

In this way, the weight for the attention score according to the present invention, which adds the bias reflecting various characteristics of the molecular structure, may be calculated according to the formula and meaning illustrated in FIG. 5. Here, A(Qk, Kk, Vk) may mean the attention weight calculated by applying the Softmax function to the attention score.

In this way, in the multi-head self-attention layer 413, when the attention operation related to the interaction between the atoms of the plurality of molecular structures is performed and the embedding vector is updated based on the operation, the updated embedding vector may be stored in the memory through the update step. Then, the updated embedding vector stored in the memory may be input to a feed-forward neural network layer 414. The feed-forward neural network layer 414 may receive the output of the multi-head attention layer 413 and additionally update the updated embedding vector. The feed-forward neural network layer 414 may update the output vector of the multi-head self-attention layer using at least one of the adjacency matrix, bond type matrix, shortest paths, and K-hop neighbors. The feed-forward neural network layer 414 may update the output vector of the multi-head self-attention layer so that the structural information on the molecular structures is included more abundantly. The output vector of the feed-forward neural network layer may be specified as a final updated embedding vector v2 input to the decoder 420.

Furthermore, the decoder 420 according to the present invention may include at least one projection layer 421. In the decoder 420, the bond prediction and atom prediction based on the plurality of molecular structures (reactants) may be performed based on the updated embedding vector v2 input to the projection layer. The bond prediction is to predict the bonds between the atoms, and the atom prediction is to predict the state change of the atoms. The decoder 420 can include a plurality of projection layers 422a and 422b. In each projection layer 422a and 422b, bond prediction 423 and atom prediction 424 may be performed.

In the decoder 420, the bond prediction may be performed by performing a dot-product using the updated embedding vector. The dot-product may be performed for each vector corresponding to each atom pair of the atoms corresponding to the updated embedding vector.

In the block 423 of the drawing, each box of x-axis and y-axis 423a and 423b conceptually represents each atom of the molecules to be reacted, and these atoms may be represented by the updated embedding vector v2. The decoder 420 may predict the bond type between each atom through the dot-product based on the information included in the updated embedding vector.

The decoder 420 may acquire inner product values for each of the plurality of bond types for each atom pair based on the dot-product. The plurality of bond types may be related to at least one of the single bond, double bond, bond formation, bond collapse, and no change.

The decoder 420 may generate a probability distribution for each of the plurality of bond types for each atom pair using the inner product values according to the dot-product. The decoder 420 may acquire a transformation matrix that predicts the change in the bonded state of each atom pair using the probability distribution.

The decoder 420 may generate the probability distribution for the plurality of bond types for each atom pair by applying the Softmax function to the inner product values acquired for the plurality of bond types for each atom pair.

The decoder 420 may generate the atomic characteristic probability distribution for each atom corresponding to the updated embedding v2 vector using a Softmax output layer. The decoder 420 may predict the atomic characteristics of the atoms corresponding to the updated embedding vector using the probability distribution. The atomic characteristics may include the charge state of the atoms that may change during the chemical reaction process of the plurality of molecular structures.

The decoder 420 may perform the dot-product between the updated embedding vectors v2 (or atom embedding vector, as illustrated in (a) of FIG. 5B) for the atoms, and calculate a score for potential bond transformation as illustrated in FIG. 5B. Here, the lowercase letter t may represent the step of the diffusion process.

This is based on a standard query-key matrix multiplication of a transformer attention mechanism, but in the present invention, the key and the query may share the same projection head. The setting that allows the key and the query to have the same projection head may require a plurality of projections to accommodate various bond types such as none, the single bond, the double bond, the triple bond, and the aromatic bond. This corresponds to the multi-head attention in the transformer, and if this is expressed in a formula, this may be represented as (c) of FIG. 5B. In this way, bond prediction may be performed in the bond prediction head. This may correspond to the first projection layer 423a illustrated. That is, the bond prediction may be performed in the first projection layer 423a. The first projection layer 423a may be composed of layers that constitute the bond prediction head.

Each bond type prediction may be viewed as a head in the multi-head attention scheme, where Softmax regularization is applied to a state such as (d) of FIG. 5B to acquire the distribution of the bond transformations for each atom pair. Here, (d) of FIG. 5B may mean the bond transformation.

In the bond prediction head, as illustrated in (c) of FIG. 5B, a score S is calculated for each bond type k, where the meanings of each symbol are as illustrated in (d) of FIG. 5B.

Meanwhile, in the bond prediction head, the probability of the bond type k of the bond transformation T according to (e) of FIG. 5D may be calculated as illustrated in (b) of FIG. 5B. In the bond prediction head, the Softmax regularization such as (f) of FIG. 5B may be applied to acquire the probability distribution of the bond transformation.

In the present invention, each bond type prediction may be understood as each head in the multi-head attention system. The decoder 420 may acquire a normalized probability distribution for the bond transformations of each atom pair by applying the Softmax normalization to the possible states of the bond transformation according to (e) of FIG. 5B. This may provide an efficient and scalable method of directly predicting the bond transformation in the embedding space.

Next, the atom prediction may be performed in the second projection layer 423b of the decoder 420. The second projection layer 423b may be composed of layers that constitute the atom prediction head. Since the features of some atoms may change during the chemical reaction, the decoder 420 according to the present invention may perform prediction on the atoms in addition to predicting the bond transformation between the atoms. The atom prediction may be performed in the second projection layer 423b. To this end, a standard Softmax output layer may be used for features of each atom. This includes the degree (i.e., the number of single bonds included in the atom) of the atom for each bond type, and the prediction may help ensure consistency between independent parallel samples of the bond transformations and between the atom transformations. In the atom prediction head, as illustrated in (c) of FIG. 5C, the probability that the feature Av of the atom v becomes a at step t of the diffusion process may be calculated. Here, (b) of FIG. 5C represents the embedding of the atom v, and (d) of FIG. 5C represents the weight matrix of the prediction head for the atomic feature a. In the atom prediction head, the probability that the feature Av of the atom becomes a may be calculated by multiplying embedding h (see (b) of FIG. 5C) of the atom v by a weight matrix W (see (d) of FIG. 5C) and then applying the Softmax function.

The decoder 420 may acquire a predicted chemical reaction product 425 (probability distribution) predicted from the chemical reaction of the plurality of molecular structures by combining the bond prediction probability and the atom prediction probability of the atoms output from the projection layers 423a and 423b. The chemical reaction product predicted from the projection layers may also be called an initial chemical reaction product.

In the present invention, the predicted chemical reaction product may be input to the graph diffusion module 430, and the graph diffusion module 430 may sample the initial chemical reaction product using the bond prediction result and the atom prediction result. The graph diffusion module 430 may stabilize the sampled initial chemical reaction product through the diffusion feedback process. The chemical reaction prediction model may acquire the final chemical reaction product stabilized through the diffusion feedback process.

In the diffusion feedback process, the graph diffusion module 430 may repeatedly evaluate each bond transformation of the initial chemical reaction product, and remove or change unstable bonds. Furthermore, the graph diffusion module 430 may evaluate the predicted bond transformation at each step repeatedly performed using the transformation probability matrix and the target transformation matrix to predict the change in the bonded state of the initial chemical reaction product, and generate the final chemical reaction product using the interpolation factor.

In the present invention, since the bond prediction is performed independently in the entire molecular graph, a method of finally acquiring a valid molecular structure is required. For example, in the prediction process, there may be cases in which the bonds to the plurality of atoms are predicted or the bonding rules are violated. In order to solve this problem, a process of denoising into a valid final state is required.

As illustrated in FIG. 5E, the stable final state may be generated through a plurality of sampling and denoising processes 432 for the predicted chemical reaction product.

FIG. 5E illustrates the diffusion process from the correct answer data (or target label (ground truth product) 440) to the initial distribution 425, and this process is based on the initial distribution derived from the dot product matrix of the bond transformation predictions between all the atom pairs. Here, the target label corresponding to the correct answer data may include actually bonded states and atomic states corresponding to the plurality of molecular structures input to the encoder. Here, the initial distribution 425 means the initial chemical reaction product 425 output from the decoder, which may correspond to the probability distribution. Meanwhile, the diffusion process to the target label is performed in the learning process in the training unit 440, and in the actual inference step, the graph diffusion module 430 for which the learning has been completed may be used. In this case, the predicted chemical reaction product 425 may be input to the graph diffusion module 430 for which the learning has been completed, and the final chemical reaction product may be acquired through the denoising process.

The training unit 440 may calculate a loss function between the final chemical reaction product output from the decoder and label data (hereinafter, referred to as "target label") including the actually bonded states and atomic states corresponding to the plurality of molecular structures, and may optimize a parameter of at least one of the encoder and the decoder to minimize the loss function.

Meanwhile, in the learning process, the target label may be sampled at random points in the interpolated space by interpolating with the initial distribution, and FIG. 5E illustrates a noise graph in which these random points are denoised with the target label. During the prediction, the results sampled from the initial distribution are updated at each step to lead to the stable final product molecule, and a similar process may be applied to the atomic features.

In the diffusion step, the predicted product may correspond to the initial distribution acquired through the dot product matrix of the bond transformation prediction for all the atom pairs in the decoder 420, as described above. This initial distribution represents the probability distribution of the bond formation between the atoms. The interpolated product (diffused product) is the target label (ground-truth product) interpolated with the initial distribution, and in the present invention, a noisy graph may be acquired by sampling the random points in this interpolated space. These random points represent the noise graph denoised with the target label. The denoised product denoises the sampled noise graph to gradually approach the target label, and this process includes several steps, and the graph may change more stably at each step. The alternative product means that in some cases, an alternative product may come out through different paths, which may have a different combination configuration from the target label.

In this way, in the present invention, the decoder 420 acquires the initial distribution, and the graph diffusion module 430 samples the initial distribution to sample the random points. The graph diffusion module 430 interpolates the target label with the initial distribution through the interpolation and denoising process to create the intermediate state. During the learning process, the intermediate state v3 may be input back to the encoder. The graph diffusion module 430 samples the random points to acquire the noisy graph, and gradually approaches the target label through the denoising process. This process may be performed repeatedly, and at least one of the encoder, decoder, and graph diffusion module may be trained in the repetitive process.

To train the method of denoising the predicted chemical reaction product 425, the graph diffusion module 430 may be configured to train a method of diffusing the target label to a previous output distribution and mapping the state in the diffused interpolation space to the final target label.

To this end, a transformation probability matrix Mt and a desired target transformation matrix T may be defined, and the transformation matrix T may be derived from the bond prediction result of the atom. The result of the bond prediction of the atom may be acquired from the decoder and may be derived from the bond prediction head described above. This may be expressed as the atom-mapped label of the reaction. The transformation matrix T may identify whether the bond is formed or broken by finding the difference between the input and output adjacency matrices, and the transformation matrix T may be expressed as in (a) of FIG. 5D. This defines the difference between the adjacency matrix of the reactants and products, and the adjacency matrix may represent the entire bond type matrix including the single/double/triple/aromatic bonds.

Meanwhile, the reactants are time-dependent because they keep changing at each diffusion step. This means that the target label also changes at each step, and in the present invention, a method of predicting when and where the difference between the reactants and the target label occurred (reaction center) and what changed (final bond configuration) when there was a difference may be trained as in (b) of FIG. 5D. A parameter at is a randomly sampled interpolation factor that determines the weight between the current transformation matrix and the target transformation matrix. During the training, the diffusion process may include acquiring the transformation probability Mt from an initial reactant set {Gr} and sampling {Gr(t+1)} to be used as the input of the next step from Mt+1. Both steps may aim to directly predict the target transformation matrix T. Therefore, the training loss may be defined as in (c) of FIG. 5D. During the prediction, the input {Gr} may be simply encoded, the prediction head may be used to acquire the probability of the bond transformation Mt, and the sample may be performed from the distribution at each step through standard categorical Softmax sampling. In the learning process of the present invention, this process may be repeated continuously and the results may be fed back until the stable configuration, in which the bond no longer changes, is acquired.

Meanwhile, in the present invention, a Top-K ranking may be applied to a plurality of stabilized products in the diffusion module 430 to derive the final chemical reaction product. This may be performed in the Top-K sampled product module of the chemical reaction prediction model 400. The Top-K ranking process may be performed in an inference stage. That is, the Top-K ranking process may be utilized to extract products with a high probability in order to provide the final product to the user. In the present invention, at least one of the Top-k sampling and Top-K ranking may be performed, which may be used to acquire more diverse results in the inference process. In the Top-k sampling, the chemical reaction prediction model may generate a plurality of samples based on the trained contents, and may diversify the distribution by adjusting a temperature parameter τ. The Top-k ranking is used to select the most likely result among the generated samples, calculate the probability of each sample, and select the result with the highest probability as the final prediction.

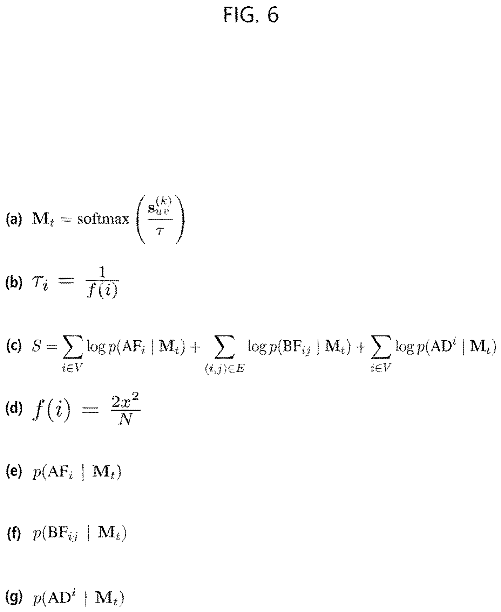

In the Top-k sampling process, when sampling the distribution Mt at time step t, logits may be readjusted by the temperature parameter τ. This may adjust the Softmax distribution illustrated in (a) of FIG. 6 more sharply or more uniformly.

Meanwhile, in order to acquire the plurality of samples, the temperature parameter τ may be used in the present invention. In the present invention, when N samples are to be extracted, τi may be set as in (b) of FIG. 6. Here, f(i) is a temperature sampling schedule, and in the present invention, f(i) may be defined as in (c) of FIG. 6. Here, x is a linspace between 0 and 1.

Next, the Top-K ranking will be described. When there are sampled candidates, a method is needed to rank the sampled candidates according to the probability. The probability may be calculated from the transformation matrix Mt. When the sampled state is considered as a sampled token for the bonds to each atom, the probability for each state may be derived from the sampled Softmax distribution Mt. Through this, the total probability for the final overall configuration (bonded state to atom) of the molecule may be assigned, and the sampled probability S may be calculated. This may be represented as in (d) of FIG. 6. Here, each of (e), (f), and (g) FIG. 6 may be derived from the Softmax distribution for the atomic feature, the bond feature, and the atomic degree. In addition, the term of (d) of FIG. 6 may ensure consistency between the predicted atomic degree and the actual degree calculated from the bond. In the present invention, since the bonds are sampled independently, the plurality of bonds may be formed on the same atom. Therefore, the actual degree of each atom is directly calculated in the sampled graph, and compared with the probability distribution of the predicted degree, and in this way, a high probability score is assigned when the actual degree of the generated molecule matches the predicted degree.

In addition, since there is no guarantee that all solutions are unique when sampling independently from the distribution, many duplicate samples may occur. Therefore, in the present invention, during the prediction, the Top-k product may be selected by taking a simple unique graph.

Meanwhile, this is described based on FIGS. 4A and 4B, which include the learning process of the chemical reaction prediction model, but as described above, the chemical reaction prediction model may have the configuration of FIGS. 4C and 4D in the inference process. In this case, since all may be understood equally except for the learning process, the detailed description will be replaced with the above description.

In the inference process, as described above and as illustrated in FIG. 3, the final chemical reaction product may be obtained by a process (S310) of receiving the information related to the plurality of molecular structures including the first molecular structure and the second molecular structure as the input of the encoder, a process (S320) of acquiring the embedding vector corresponding to the first molecular structure and the second molecular structure using the information related to the plurality of molecular structures, in the embedding layer of the encoder, a process (S330) of performing the attention operation related to the interaction between the atoms of the first molecular structure and the second molecular structure and updating the embedding vector based on the operation, in the multi-head self-attention layer, and a process (S340) of storing the updated embedding vector through the update step in the memory and inputting the updated embedding vector stored in the memory to the decoder, a process (S350) of performing the bond prediction and the atom prediction predicted as the chemical reaction of the first molecular structure and the second molecular structure using the updated embedding vector in the decoder, and a process (S360) of acquiring the final chemical reaction product predicted from the chemical reaction of the first molecular structure and the second molecular structure using the result of the bond prediction and the result of the atom prediction.

The process for acquiring the final chemical reaction product may be performed through the answer generation system 100 as illustrated in FIG. 1. In this case, the answer generation system 100 may receive the user query, and the user query may include the information on the molecular structure that is the target of the chemical reaction prediction.

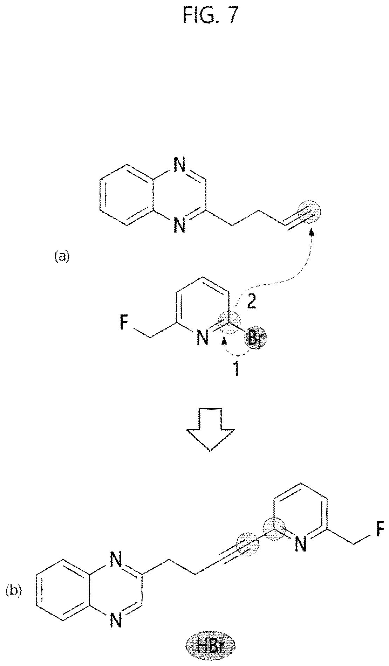

In the chemical reaction prediction model 400 according to the present invention, as illustrated in (b) of FIG. 7, the chemical reaction product may be acquired through the bond prediction and atom prediction for the plurality of molecular structures that are the targets of the chemical reaction as illustrated in (a) of FIG. 7.

As described above, according to the chemical reaction prediction system and the control method thereof, and the learning method of a chemical reaction prediction system according to the present invention, by modeling the movement of electrons through the graph structure of molecules, it is possible to better understand the chemical reaction mechanism and accurately predict the results of the chemical reaction.

In addition, according to the chemical reaction prediction system and the control method thereof according to the present invention, and the learning method of a chemical reaction prediction system, by providing the chemical reaction prediction model that can accurately predict the results of the chemical reaction and better understand the chemical reaction mechanism, it is possible for a user to reduce the time and cost required for experiments. Accordingly, it is possible to reduce the research and development costs and shorten the time to market for new products.

Meanwhile, according to the chemical reaction prediction system, the control method thereof, and the learning method of a chemical reaction prediction system according to the present invention, by performing both the input of the molecular structure and the output of the predicted product in the graph space, it is possible to perform the forward reaction prediction regardless of the SMILES permutation and order.

Furthermore, according to the chemical reaction prediction system, the control method thereof, and the learning method of a chemical reaction prediction system according to the present invention, by simultaneously sampling multiple interdependent transformations that occur in parallel within the molecular graph, it is possible to secure the consistency between the transformations.

Furthermore, according to the chemical reaction prediction system, the control method thereof, and the learning method of a chemical reaction prediction system according to the present invention, in order to solve problems that may occur due to the symmetrical structure, by breaking the symmetry and forming the valid output structure by including the noise or sampling mechanisms, it is possible to prevent the occurrence of the invalid configuration.

Meanwhile, the present invention described above may be implemented as a program that is executed by one or more processes on a computer and can be stored on a computer-readable medium (or recording medium).

Furthermore, the present invention described above can be implemented as a computer-readable code or instruction on a medium in which a program is recorded. The present invention may be provided in the form of a program.

Meanwhile, the computer-readable medium may include all kinds of recording devices in which data that may be read by a computer system are stored. Examples of the computer-readable medium include a hard disk drive (HDD), a solid state disk (SSD), a silicon disk drive (SDD), a read only memory (ROM), a random access memory (RAM), a compact disk (CD)-ROM, a magnetic tape, a floppy disk, an optical data storage device, and the like.

Furthermore, the computer-readable medium may include a storage and may be a server or a cloud storage that an electronic device may access through communication. In this case, the computer may download the program according to the present invention from the server or cloud storage through wired or wireless communication.

Furthermore, in the present invention, the computer described above is an electronic device equipped with a processor, that is, a central processing unit (CPU), and the type of electronic device is not particularly limited.

Meanwhile, the above-described detailed description is to be interpreted as being illustrative rather than being restrictive in all aspects. The scope of the present invention is to be determined by reasonable interpretation of the claims, and all modifications within an equivalent range of the present invention fall in the scope of the present invention.

## Claims

1. A chemical reaction prediction method performed by cooperation of a memory and a processor, the chemical reaction prediction method comprising:
receiving information related to a plurality of molecular structures as input to an encoder;
acquiring an embedding vector corresponding to the plurality of molecular structures using the information related to the plurality of molecular structures in an embedding layer of the encoder;
performing an attention operation related to interaction between atoms of the plurality of molecular structures in a multi-head self-attention layer and updating the embedding vector based on the operation;
storing the embedding vector updated through the updating in the memory and inputting the updated embedding vector stored in the memory to a decoder;
performing bond prediction and atom prediction predicted as a chemical reaction of the plurality of molecular structures using the updated embedding vector in the decoder; and
acquiring a final chemical reaction result predicted from the chemical reaction of the plurality of molecular structures using a result of the bond prediction and a result of the atom prediction.

2. The chemical reaction prediction method of claim 1, wherein the acquiring of the chemical reaction product includes
sampling an initial chemical reaction product using the result of the bond prediction and the result of the atom prediction,
stabilizing the sampled initial chemical reaction product through a diffusion feedback process, and
acquiring the final chemical reaction result stabilized through the diffusion feedback process.

3. The chemical reaction prediction method of claim 1, further comprising acquiring a molecular graph for the plurality of molecular structures by converting atoms into nodes and bonds between atoms into edges based on the plurality of molecular structures,
wherein the plurality of molecular structures include a first molecular structure and a second molecular structure, and
the acquiring of the molecular graph further includes
acquiring a first molecular graph including nodes and edges corresponding to the first molecular structure by converting atoms constituting the first molecular structure into nodes and a bond relationship between atoms constituting the first molecular structure into edges using a pre-specified graph transformation algorithm, and
acquiring a second molecular graph including nodes and edges corresponding to the second molecular structure by converting atoms constituting the second molecular structure into nodes and a bond relationship between atoms constituting the second molecular structure into edges using the pre-specified graph transformation algorithm.

4. The chemical reaction prediction method of claim 3, wherein the information related to the plurality of molecular structures includes information on the nodes and edges corresponding to the first molecular structure and information on the nodes and edges corresponding to the second molecular structure, and
the embedding vector includes at least one of information on an atom type, an atom charge, the number of hydrogens, the number of radical electrons, and a degree of the node corresponding to the first molecular structure and the node corresponding to the second molecular structure.

5. The chemical reaction prediction method of claim 4, wherein, in the updating of the embedding vector, different biases are added to an attention score operated by the multi-head self-attention layer according to a bond type between the nodes constituting the first molecular graph and the second molecular graph.

6. The chemical reaction prediction method of claim 5, wherein the bond type includes a single bond type, a double bond type, a triple bond type, and an aromatic bond type.

7. The chemical reaction prediction method of claim 5, further comprising extracting, using the nodes and edges corresponding to the first molecular graph and the nodes and edges corresponding to the second molecular graph, at least one of an adjacency matrix, a bond type matrix, a shortest paths matrix, and K-hop neighbors corresponding to each of the first molecular graph and the second molecular graph,
wherein the adjacency matrix includes information on direct connection between the nodes constituting the first molecular graph and the second molecular graph,
the bond type matrix includes information on a bond type between the nodes constituting the first molecular graph and the second molecular graph,
the shortest paths matrix includes information on a shortest path length between the nodes constituting the first molecular graph and the second molecular graph, and
the K-hop neighbors include information on neighboring nodes within a K step for each of the nodes constituting the first molecular graph and the second molecular graph.

8. The chemical reaction prediction method of claim 7, wherein in the updating of the embedding vector,
an output vector of the multi-head self-attention layer is added to a feed-forward neural network layer,
in the feed-forward neural network layer, the output vector of the multi-head self-attention layer is updated using at least one of the adjacency matrix, the bond type matrix, the shortest paths matrix, and the K-hop neighbors, and
the output vector of the feed-forward neural network layer is specified as the updated embedding vector.

9. The chemical reaction prediction method of claim 1, wherein the bond prediction is performed by performing a dot-product using the updated embedding vector, and
the dot-product is performed for each vector corresponding to each atom pair of atoms corresponding to the updated embedding vector.

10. The chemical reaction prediction method of claim 9, wherein the performing of the bond prediction includes acquiring an inner product value for each of plurality of bond types for each atom pair based on the dot-product, and
the plurality of bond types are related to at least one of a single bond, a double bond, a bond formation, a bond collapse, and no change.

11. The chemical reaction prediction method of claim 10, wherein the performing of the bond prediction further includes
generating a probability distribution for each of the plurality of bond types for each atom pair using the inner product value according to the dot-product, and
acquiring a transformation matrix that predicts a change in a bonded state of each atom pair using the probability distribution.

12. The method of claim 11, wherein in the generating of the probability distribution, for each atom pair, a Softmax function is applied to the inner product values acquired for the plurality of bond types for each atom pair to generate the probability distribution for the plurality of bond types for each atom pair.

13. The chemical reaction prediction method of claim 1, wherein the performing of the atom prediction further includes:
generating an atomic characteristic probability distribution of each atom corresponding to the updated embedding vector using a Softmax output layer; and
predicting atomic characteristics of the atoms corresponding to the updated embedding vector using the probability distribution, and
the atomic characteristics include charge states of the atoms changeable during a chemical reaction process of the plurality of molecular structures.

14. The chemical reaction prediction method of claim 2, wherein in the diffusion feedback process, each bond transformation of the initial chemical reaction product is repeatedly evaluated and an unstable bond is removed or changed.

15. The chemical reaction prediction method of claim 14, wherein in the diffusion feedback process, in order to predict a change in a bonded state of the initial chemical reaction product, predicted bond transformation is evaluated at each repeatedly performed step using a transformation probability matrix and a target transformation matrix, and the final chemical reaction result is generated using an interpolation factor.

16. A learning method for a chemical reaction prediction method performed by cooperation of a memory and a processor, the chemical reaction prediction method comprising:
receiving information related to a plurality of molecular structures as input to an encoder;
acquiring a molecular graph using atoms as nodes and bonds as edges based on the plurality of molecular structures;
acquiring an embedding vector corresponding to the molecular graph using the information related to the plurality of molecular structures in an embedding layer of the encoder;
performing an attention operation related to interaction between atoms of the plurality of molecular structures in a multi-head self-attention layer and updating the embedding vector based on the operation;
storing the embedding vector updated through the updating in the memory and inputting the updated embedding vector stored in the memory to a decoder;
performing bond prediction and atom prediction predicted as a chemical reaction of the plurality of molecular structures using the updated embedding vector in the decoder;
acquiring a final chemical reaction product predicted from the chemical reaction of the plurality of molecular structures using a result of the bond prediction and a result of the atom prediction;
calculating a loss function between the final chemical reaction result and label data including an actually bonded state and an atomic state corresponding to the plurality of molecular structures; and
optimizing at least one parameter of the encoder and the decoder to minimize the loss function.

17. A chemical reaction prediction system comprising:
a memory;
an encoder;
a decoder; and
at least one a processor,
wherein the encoder receives information related to a plurality of molecular structures, and acquires a molecular graph using atoms as nodes and bonds as edges based on the plurality of molecular structures,
acquires an embedding vector corresponding to the molecular graph in an embedding layer of the encoder, and
performs an attention operation related to interaction between atoms of the plurality of molecular structures in a multi-head self-attention layer of the encoder and updates the embedding vector based on the operation,
the processor stores the embedding vector updated through the updating in the memory, and inputs the updated embedding vector stored in the memory to the decoder, and
the decoder performs bond prediction and atom prediction predicted as a chemical reaction of the plurality of molecular structures using the updated embedding vector, and
acquires a final chemical reaction product predicted from the chemical reaction of the plurality of molecular structures using a result of the bond prediction and a result of the atom prediction.

18. A program stored on a computer-readable recording medium, executable by one or more processes on an electronic device, the program comprising instructions to execute:
receiving information related to a plurality of molecular structures as input to an encoder;
acquiring a molecular graph using atoms as nodes and bonds as edges based on the plurality of molecular structures;
acquiring an embedding vector corresponding to the molecular graph in an embedding layer of the encoder;
performing an attention operation related to interaction between atoms of the plurality of molecular structures in a multi-head self-attention layer and updating the embedding vector based on the operation;
storing the embedding vector updated through the updating in a memory and inputting the updated embedding vector stored in the memory to a decoder;
performing bond prediction and atom prediction predicted as a chemical reaction of the plurality of molecular structures using the updated embedding vector in the decoder; and
acquiring a final chemical reaction product predicted from the chemical reaction of the plurality of molecular structures using a result of the bond prediction and a result of the atom prediction.
